# EUROPEAN PATENT SPECIFICATION

(11) **EP 0 643 626 B1**
(45) Date of publication and mention of the grant of the patent: **27.02.2002**
(21) Application number: 92919443.9
(22) Date of filing: 26.08.1992
(51) Int. Cl.: B01J 31/00, C07F 9/00, C07F 13/00, C07F 11/00, C07F 15/00, C07D 493/00, C07D 301/06, C07D 301/12, C07D 301/14, C07D 305/00, C07D 493/04, B01J 31/18, C07C 315/02, C07D 305/14, C07D 303/48

(54) **METHOD OF PRODUCING EPOXYCHROMANS WITH A CHIRAL CATALYST**
HERSTELLUNG VON EPOXCHROMANEN MIT EINEM CHIRALEN KATALYSATOR
PROCEDE DE PRODUCTION D'EPOXYCHROMANNES AVEC UN CATALYSEUR CHIRAL

(30) Priority: 26.08.1991 US 749460; 16.12.1991 US 809446
(43) Date of publication of application: 22.03.1995
(73) Proprietor: Research Corporation Technologies, Inc, Tucson, Arizona 85711-3335 (US)
(72) Inventor: JACOBSEN, Eric, N., Boston, MA 02115 (US); ZHANG, Wei, Maple Glen, PA 19002 (US); DENG, Li, Brookline, MA 02146 (US)
(74) Representative: Vossius, Volker, Dr.
(86) International application number: US9207261
(87) International publication number: WO9303838

(56) References cited:
- WO-A-91/14694
- US-A- 4 924 011
- US-A- 5 015 744
- TETRAHEDRON LETTERS, vol.32, no.38, 16 September 1991 pages 5055 - 5058 NAM HO LEE ET AL
- Journal of American Chemical Society, Vol. 110, issued 1988 (ACS), DENIS et al., "A Highly Efficient, Practical Approach to Natural Taxol", see pages 5917-5919.
- Tetrahedron, Vol. 46, No. 11, issued 1990 (Pergamon Press), HOENIG et al., "Chemo-enzymatic Synthesis of all Isomeric 3-Phenylserines and-Isoserines", see pages 3841-3850.
- Journal of American Chemical Society, Vol. 110, issued 1988 (ACS), YOON et al., "Catalysis of Alkene Oxidation by Nickel Salen Complexes Using NaOC1 under Phase-Transfer Conditions", see pages 4087-4089.
- Journal of American Chemical Society, Vol. 112, No. 7, issued 28 March 1990 (ACS), ZHANG et al., "Enantioselective Epoxidation of Unfunctionalized Olefins Catalyzed by (Salen) Manganese Complexes", pages 2801-2803, see entire document.
- Journal of American Chemical Society, Vol. 113, No. 18, issued 28 August 1991 (ACS), JACOBSEN et al., "Highly Enantioselective Epoxidation Catalysts Derived from 1,2-Diaminocyclohexane", pages 7062-4, see entire document.
- Bulletin Chemical Society of Japan, Vol. 56, No. 1, issued January 1983, KANATOMI, "The Dehydrogenation of Nickel (II) Chelates of rac- and meso-2,2'-((1,2-Diphenylethylene)bis(iminom ethylene)diphenol and Related compounds", see pages 99-104.
- Chemistry Letters, issued 1986 (The Chemical Society of Japan), NAKAJIMA et al., "Asymmetric Oxidation of Sulfides to Sulfoxides by Organic Hydroperoxides with Optically Active Schiff Base - Oxovanadium (IV) Catalysts", page 1483-6, see entire document.
- Journal of American Chemical Society, Vol. 113, No. 17, issued 14 August 1991 (ACS), JACOBSEN et al., "Electronic Tuning of Asymmetric Catalysts", pages 6703-4, see entire document.

## Description

The present invention relates to the field of asymmetric catalysis. More particularly, the invention relates to the field of organometallic catalysts useful for enantioselectively epoxidizing prochiral olefins.

Several advances in catalysis of asymmetric group transfer have occurred in recent years. One such advance has been the discovery by K.B. Sharpless et al. of the epoxidation of allylic alcohols which provides access to enantiomerically pure synthetic building blocks. Unformmately, Sharpless catalysis requires the presence of a specific functional group, namely an allylic alcohol, on the olefin to be epoxidized. Naturally, this requirement severely limits the variety of olefins which can be so epoxidized.

Some success has been achieved in asymmetric catalysis of unfunctionalized olefins. For example, K.B. Sharpless reported in 1988 that certain cinchona alkaloid derivatives were effective ligands in the osmium-catalyzed asymmetric dihydroxylation of trans-stilbene and various other olefins. This method provides a practical route to certain chiral diols, although cis olefins afford poor results.

Aside from the catalysts disclosed herein, it is believed that there currently exists no practical catalytic method for the asymmetric epoxidation of unfunctionalized olefins. Some progress has been made in this area through the use of chiral porphyrin complexes. In particular, J.T. Groves et al. reported in 1983 the asymmetric epoxidation of styrene by a chiral iron porphyrin catalyst. Unformmately, the Groves system suffers several disadvantages, namely, the porphyrin catalyst is relatively difficult to prepare, oxidant proceeds to low substrate conversion, is limited to styrene derivatives, and achieves enantiomeric excess (ee) values of less than about 50 percent.

### Epoxychroman Synthesis

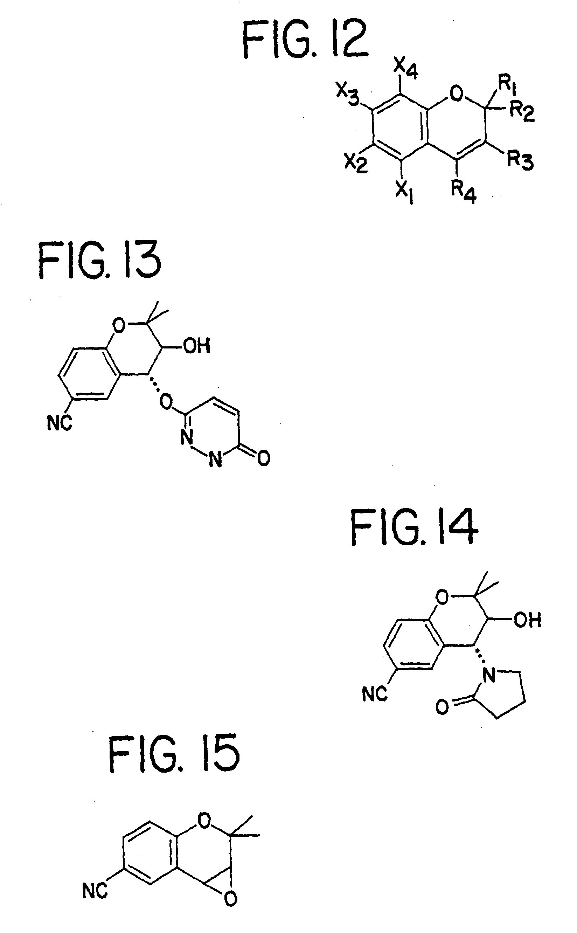

Given the broad synthetic utility of epoxides, a simple, reliable, and practical procedure for asymmetric epoxidation of simple olefins is clearly desirable. One class of chiral epoxide with synthetic utility is the group of compounds generally known as epoxychromans, or epoxides of derivatives of chromene. For example, the epoxide of 6-cyano-2,2-dimethylchromene has been found to be useful in the synthesis of a compound known as cromakalim. Two variations of cromakalim are shown in FIGURES 13 and 14. Both of these are believed to be potassium channel activators and have shown considerable promise as antihypertensive drugs.

As can be seen in FIGURES 13 and 14, the cromakalim compounds have two enantiomers. It is currently believed that only one of these enantiomers, namely the 3S, 4R enantiomer, possesses the antihypertensive activity. Consequently, a method of making a more enantiomerically pure epoxide of the precursor chromene derivative is highly desirable.

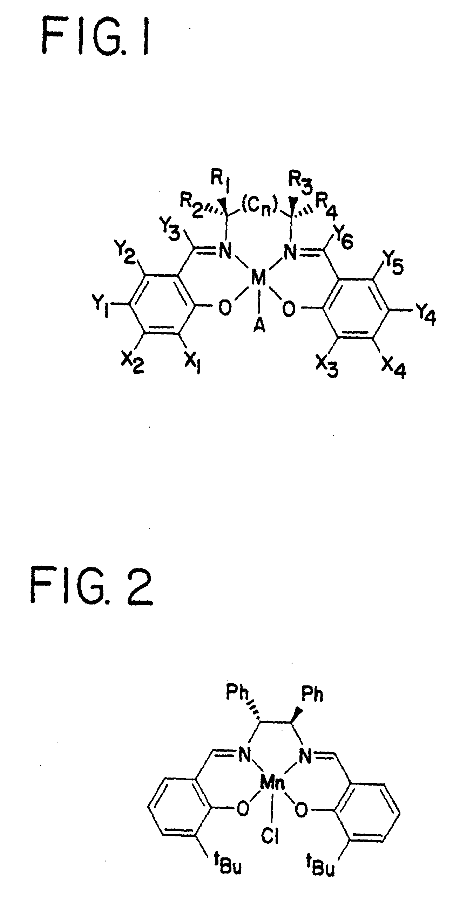

The article in the Journal of American Chemical Society, Vol. 113, No. 17, issued 14 August 1991 (ACS), by JACOBSEN et al., "Electronic Tuning of Asymmetric Catalysts", pages 6703-6704 discloses epoxidation of 2,2-dimethylchromene with Mn-salen catalysts, prepared from 5-substituted tert.butyl salicylaldehyde derivatives with 1,2-diaminocyclohexane (2a-e) or 1,2-diamino-1,2-diphenylethane (1a-e) (page 6703: right column: scheme I and 2^{nd} paragraph-4^{th} paragraph, 1^{st} sentence; page 6704: figure 1, (4)).

However, catalysts (2a-e) do not contain blocking substituents at the "side" positions and this article teaches that the rate of epoxidation is increased by electron-withdrawing groups at the "side" positions of the catalyst (page 6704: left column, last paragraph).

WO91/14694 which is an elder European right according to Article 54(3) and (4) EPC for the contracting states of AT, BE, CH, DE, DK, ES, FR, GB, GR, IT, LU, NL and SE discloses a method for the epoxidation of 2,2-dimethylchromene with a catalyst as defined in the present invention wherein the anion A=Cl and R1=R4=H, or either R1 or R4 or both are primary alkyls.

The present invention relates to a method of enantioselectively epoxidizing a chromene derivative to an epoxychroman with a chiral catalyst comprising the steps of:
oxidizing a chromene derivative having the general formula: 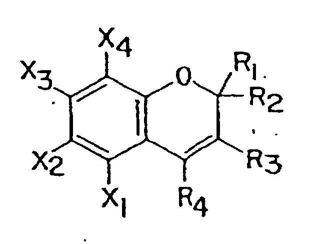
wherein R1, R2, R3, R4, X1, X2, X3 and X4 are each selected from the group consisting of hydrogen, aryls, primary alkyls, secondary alkyls, tertiary alkyls, and hetero atoms, and wherein no more than one of R1 and R2 are hydrogen;
with an oxygen atom source; in the presence of a chiral catalyst having the general formula: 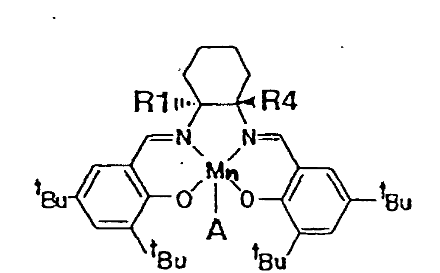
where A is an anion and
where R1 and R4 are trans to each other and at least one of R1 and R4 is selected from the group consisting of primary alkyls and hydrogen,
with the proviso that the chromene derivative is not 2,2-dimethylchromene when the anion A of the catalyst is Cl and R1 = R4 = H or either R1 or R4 or both are primary alkyls for the contracting states AT, BE, CH, DE, DK, ES, FR, GB, GR, IT, LU, NL and SE.

In accordance with the method aspect of the invention, a chromene derivative, an oxygen atom source, and the chiral catalyst used in the invention are reacted under such conditions and for such time as is needed to epoxidize said chromene derivative.

In accordance with a preffered embodiment of this invention, a pyridine-N-oxide derivative is used. Preferably, 4-phenylpyridine-N-oxide or 4-t-butylpyridine-N-oxide is used. More preferably, 4-phenylpyridine-N-oxide is used.

The present invention provides a method of producing an epoxychroman using a chiral catalyst. In accordance with this method, a chromene derivative, an oxygen atom source, and a chiral catalyst from those described below are reacted under such conditions and for such time as is needed to epoxidize said chromene derivative.

The chromene derivative used in the present method has the following structure: 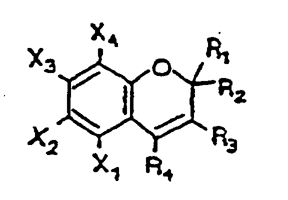 wherein R1, R2, R3, R4, X1, X2, X3, and X4 are each selected from the group consisting of hydrogen, aryls, primary alkyls, secondary alkyls, tertiary alkyls, and hetero atoms, and wherein no more than one of R1 and R2 are hydrogen.

The enantioselective method of producing an epoxychroman has provided certain advantages. First, the preferred catalysts used in the invention show remarkable enantioselectivity in catalyzing the epoxidation of chromene derivatives. See Example 2 below, where an ee of 97% was obtained with the 6-cyano-2,2-dimethylchromene and the most preferred catalyst shown below.

Second, the present invention provides an effective and concise route to epoxychromans with very high enantioselectivity. Enantiomerically enriched epoxychromans are valuable intermediates for the synthesis of chiral 3,4-disubstituted chromans.

Third, the method is effective with a wide variety of substituted chromene derivatives.

Fourth, the catalysts used in the present invention are relatively easy to synthesize, particularly as compared to the porphyrin systems disclosed in the prior art.

The catalysts used in the method of the present invention belong to the fourth group of exemplary groups one to four of catalysts illustrated in the present description herein below.

### BRIEF DESCRIPTION OF THE DRAWINGS

FIGURE 1 shows the generalized 2-dimensional structure of a catalyst of the first group.
FIGURE 2 shows the 2-dimensional structure of the most preferred catalyst of the first group.
FIGURE 3 shows the generalized 2-dimensional structure of the catalyst of the second group.
FIGURE 4 is a computer generated 3-dimensional view of the most preferred catalyst of the first group in its proposed oxo-intermediate state.
FIGURES 5A-5C are views similar to FIGURE 4 illustrating the steric hindrance believed to be responsible for the high enantioselectivity observed in the epoxidation of one of the preferred substrates by the preferred catalysts of the first group.
FIGURE 6 shows the generalized 2-dimensional structure of the catalyst of the third group.
FIGURE 7 shows the generalized 2-dimensional structure of a catalyst of the fourth group.
FIGURE 8 shows the 2-dimensional structure of the preferred catalyst of the fourth group.
FIGURE 9 is a 2-dimensional representation of the theorized favored approach of a prochiral olefin to a preferred catalyst of the first group.
FIGURE 10 is a 2-dimensional representation of the theorized favored approach of a prochiral olefin to a preferred catalyst of the second group.
FIGURE 11 shows 2-dimensional structures for various embodiments of the present invention with the numbering system used in Example 1.
FIGURE 12 shows the structure of the chromene derivatives used in the present method.
FIGURE 13 shows the structure of the 3S, 4R enantiomer of one cromakalim compound.
FIGURE 14 shows the structure of the 3S, 4R enantiomer of another cromakalim compound.
FIGURE 15 shows the structure of 6-cyano-2,2-dimethyl-3,4-epoxychroman.

As noted above, the present invention is a method of using chiral catalysts for enantioselectively epoxidizing chromene derivatives.

### The First Group of Catalysts

FIGURE 1 shows the structure of the first group of chiral catalysts.

The preferred catalysts are salen derivative-based complexes of a metal ion. The term "salen" is used herein to refer to those ligands typically formed through a condensation reaction of two molecules of a salicylaldehyde derivative with one molecule of a diamine derivative. While salen ligands are formed from ethylenediamine derivatives, propyl and butyl diamines may also be used to give analogous salpn and salbn derivatives. Salen derivatives arc preferred and their general structure is shown in FIGURE 1. A salen derivative where n is 0 is shown in FIGURE 2.

As seen in FIGURE 1, the two nitrogens and the two oxygens are oriented toward the center of the salen ligand and thus provide a complexing site for the transition metal ion M. Preferably, this metal ion is selected from the group consisting of Mn, Cr, Fe, Ni, Co, Ti, V, Ru, and Os. More preferably, the transition metal ion is selected from the group consisting of Mn, Cr, Fe, Ni, and Co. Most preferably, the metal ion is Mn.

The selection of the anion, A, is not seen to be critical to the performance of the catalyst. Preferably, the anion is selected from the group consisting of PF₆, (aryl)₄, BF₄, B(aryl)₄, halide, acetate, triflate, tosylate, with halide or PF₆ being more preferred, and chloride being most preferred.

FIGURE 1 also shows the many sites available for substitution on the salen ligand. Of these sites, it is believed that R1, R2, R3, R4, and X1, X2, X3, X4, Y3 and Y6 are the most important in this first group of catalysts.

According to the first group, at least one of the X1 and X2 sites, and at least one of the X3 and X4 sites include a substituent selected from the group consisting of secondary or tertiary alkyl groups, aryl groups, silyl groups, and alkyl groups bearing heteroatom substituents such as alkoxy or halide. For reasons to be discussed below, these will be referred to as "blocking" substituents. Preferably, it is the X1 and X3 sites which bear one of these blocking substituents. More preferably, X1 and X3 bear the same substituent, which substituent is most preferably a tertiary alkyl group, such as tertiary butyl. Preferably, when X1 and X3 bear the blocking substituent, then X2 and X4 can be selected from a group of non-blocking substituents such as H, CH₃, C₂H₅, and primary alkyls, most preferably, H. Alternatively, either three or four of X1, X2, X3, and X4 can be selected from the group of blocking substituents.

According to this first group, at least one and no more than two of R1, R2, R3 and R4 are selected from a group consisting of H, CH₃, C₂H₅, and primary alkyls. For convenience, and consistent with the present theory to be discussed below, this group will be referred to as the non-blocking group. If R1 is selected from the non-blocking group, then R2 and R3 arc selected from the blocking group. If R2 is selected from the non-blocking group, then R1 and R4 are selected from the blocking group. Likewise, if R3 is selected from the non-blocking group, then R1 and R4 are selected from the blocking group. Finally, if R4 is selected from the non-blocking group, then R2 and R3 are selected from the blocking group.

Stated in other terms, this first group requires that, of the four sites available for substitution on the two carbon atoms adjacent to nitrogen, either one or two of these will include a substituent from the non-blocking group. The first group also requires that the remaining sites include a substituent from the blocking group. In addition, it is a requirement that there not be two non-blocking substituents on the same carbon, and that there not be two non-blocking substituents on the same side on the two different carbons, i.e. not cis across the nitrogen.

Stated in yet another way, if there is only one non-blocking substituent, that non-blocking substituent can be on any one of the four substitution sites, R1, R2, R3, and R4, and the other three sites must include a blocking substituent. If, on the other hand, there are two non-blocking substituents, then they must be on different carbon atoms, and they must be trans to each other.

Preferably, the non-blocking substituent is either hydrogen or methyl, but most preferably, hydrogen. Preferably, the blocking substituent is either a phenyl group or a tertiary butyl group, but most preferably a phenyl group.

The substituents on the Y3 and Y6 sites affect the conformation of the ligand and thus have an influence on enantioselectivity in the epoxidation. Preferably, Y3 and Y6 arc hydrogen, methyl, alkyl, or aryl. More preferably, they are hydrogen or methyl. Most preferably, they are hydrogen.

The Y1, Y2, Y4, and Y5 sites are seen to be less critical. Preferably, these sites are occupied by hydrogen, although these sites may also be occupied by substituents independently selected from the group consisting of hydrogen, halides, alkyls, aryls, alkoxy groups, nitro groups.

FIGURE 2 shows the structure of the most preferred catalyst of this first group of catalysts. As can be seen, the most preferred substituent at X1 and X3 is a t-butyl group. Also, it is most preferred for the R1 and R4 sites to have the same blocking group, namely a phenyl group. In addition, it is most preferred to have the R2 and R3 sites occupied by a hydrogen. Finally, it is most preferred that the X2, X4, Y1, Y2, Y3, Y4, Y5, and Y6 sites are also all occupied by a hydrogen.

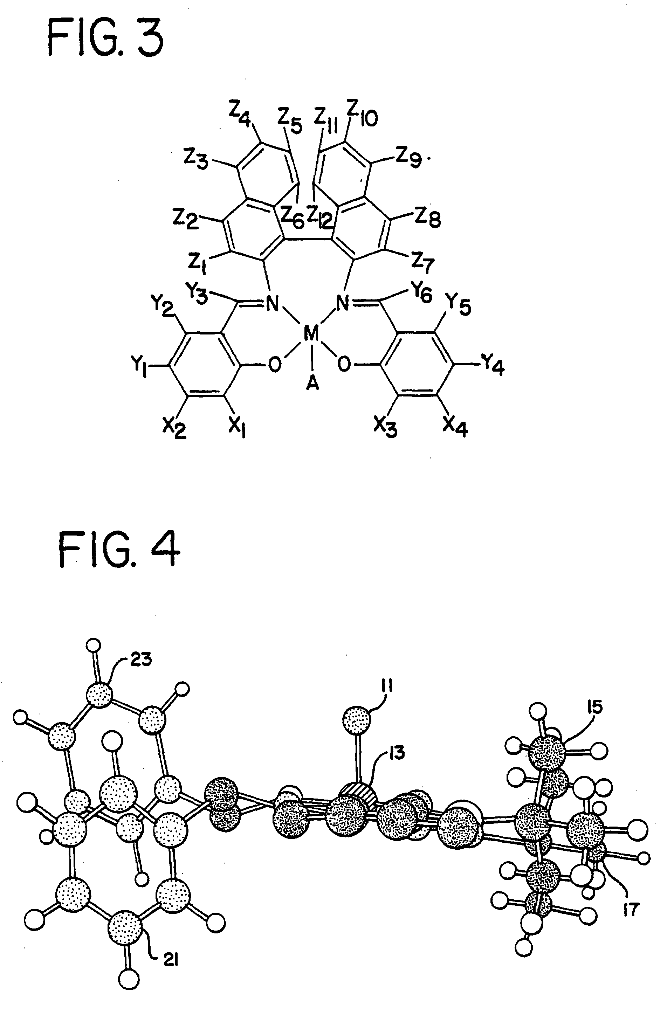

While not wishing to be bound by any particular theory, the following mechanism has been proposed to explain the remarkable enantio-selectivity of the first group of catalysts. Referring to FIGURE 4, which is a 3-dimensional view of the R,R enantiomer of the most preferred catalyst in its proposed oxo-intermediate state, it is seen that, with important exceptions, the salen ligand assumes a generally planar conformation with the oxygen atom 11 being complexed with the Manganese ion 13 and aligned on an axis generally perpendicular to this plane. The exceptions are the tert-butyl blocking groups attached at the X1 and X3 sites 15 and 17 respectively, and the phenyl blocking groups attached at the R1 and R3 sites 21 and 23, respectively. Although hard to depict in two dimensions, the phenyl blocking group 23 at R4 is behind the phenyl blocking group 21 at R1, while the R4 phenyl blocking group 23 is substantially above the plane of the catalyst and the R1 phenyl blocking group 21 is substantially below the plane of the catalyst.

FIGURES 5A-5C show the different transition orientations possible for a cis-disubstituted olefin, namely cis-methylstyrene, which are possible during epoxidation of the double bond.

FIGURE 5A shows the favored orientation, i.e. the orientation with the least steric hindrance between the olefin and the blocking groups of the catalyst. This orientation results when the double bond approaches the oxygen atom from the front (as shown). This orientation results in the formation of the 1R,2S enantiomer of the cis-β-methylstyreneoxide.

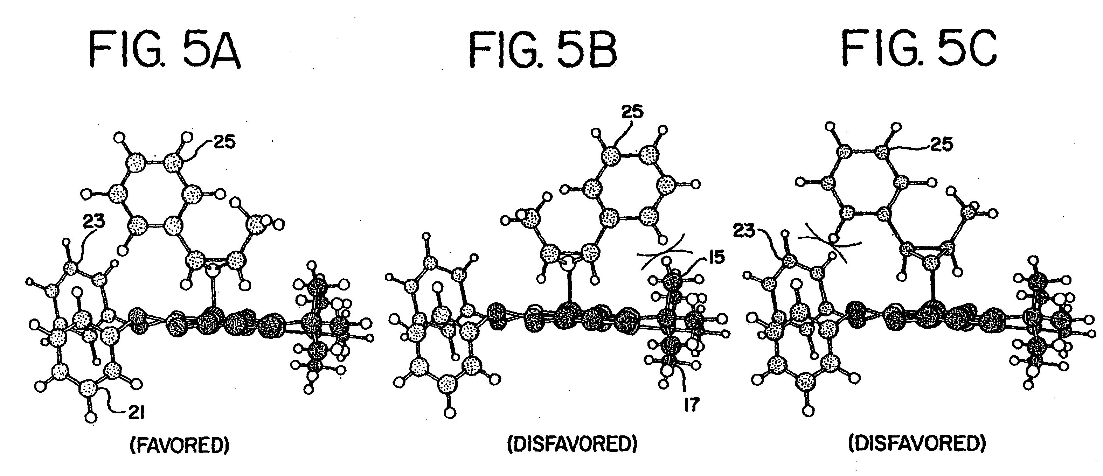

FIGURE 5B shows an orientation wherein methylstyrene has been rotated 180 degrees thus bringing the phenyl group of the styrene closer to the t-butyl groups 15 and 17 at the X1 and X3 positions. It is expected that steric hindrance between the phenyl group of the styrene 25 and the t-butyl groups 15 and 17 would disfavor this orientation.

FIGURE 5C shows an orientation resulting from the double bond approaching the oxygen atom from behind (as shown). This orientation results in the formation of the 1S,2R enantiomer of the cis-β-methylstyrene oxide. In this orientation the phenyl group of the styrene 25 is closer to the phenyl group 23 on the R4 site. Steric hindrance between these two phenyl groups would thus disfavor this approach from behind the oxygen atom, and thus disfavor synthesis of the 1S,2R enantiomer.

In contrast, the orientation shown in FIGURE 5A results from an approach from the front, i.e. the side where the R1 phenyl group 21 is below the plane of the catalyst, and thus not in the way. For this reason, the approach depicted in FIGURE 5A is sterically favored, and thus synthesis of the 1R,2S enantiomer is favored.

It should be borne in mind that, although the above-described mechanism accurately predicts the high degree of enantioselectivity observed in the catalysts of the first group, the mechanism is at present only a theory. As such, the proposed mechanism should in no way limit the scope of the present invention as defined by the appended claims.

It is noted that synthesis of the 1S,2R enantiomer of the cis-β-methylstyrene oxide is favored by using the S,S enantiomer of the catalyst.

It is also noted that this most preferred catalyst has C₂ symmetry, i.e. it is identical when rotated 180 degrees. Consequently, whether the oxygen atom is aligned on the top of the catalyst as shown, or the bottom of the catalyst, the result is exactly the same.

In alternative embodiments, the catalyst has only approximate C₂ symmetry. In particular, as per the rules described above, the groups are positioned on R1-R4 so that when rotated 180°, the blocking groups are in the same place and the non-blocking groups are in the same place. Consequently, the enantioselectivity of the catalyst is maintained because the oxygen can be complexed to either side of the catalyst while achieving roughly the same steric hindrances which favor the approach of the prochiral olefin from one side.

In other alternative embodiments, the catalyst has only one non-blocking group. As a result, there is a favored approach only when the oxygen is aligned on one side of the catalyst. Thus, the enantioselectivity of the catalyst is maintained.

### The Second Group of Catalysts

In accordance with the second group, the chiral catalyst is made with a binaphthyl diamine and has the following general structure (see also FIGURE 3):

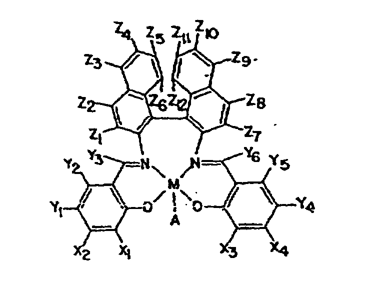

In this binaphthyl catalyst, the transition metal ion M and the anion A are preferably selected from the same group as that discussed above with FIGURE 1. Also as above, it is required that at least one of X1 and X2 together with at least one of X3 and X4 are occupied by a group selected from the group of blocking substituents consisting of secondary or tertiary alkyl groups, aryl groups, silyl groups, and alkyl groups bearing heteroatom substituents such as alkoxy or halide. Preferably, it is the X1 and X3 sites which bear one of these substituents. More preferably, X1 and X3 bear the same substituent, which substituent is most preferably a tertiary alkyl group, such as tertiary butyl.

The substituents on the Y3 and Y6 sites affect the conformation of the ligand and thus have an influence on enantioselectivity in the epoxidation. Preferably, Y3 and Y6 are hydrogen, methyl, alkyl, or aryl. More preferably, they are hydrogen or methyl. Most preferably, they are hydrogen.

The substituents Z1 and Z2 affect the differentiation between the faces of the proposed metal oxo and thus have an influence on enantioselectivity in the epoxidation. Preferably, Z1 and Z2 are hydrogen, ethyl, akyl, silyl, or aryl. More preferably, they are alkyl or aryl groups.

The Y1, Y2, Y4, and Y5 sites on the catalyst of this second group are also seen to be less critical. As above, these sites are preferably occupied by hydrogen, although these sites may also be occupied by substituents independently selected from the group consisting of hydrogen, halides, alkyls, aryls, alkoxy groups, nitro groups.

As can be visualized, this binaphthyl alternative catalyst effects the same enantioselectivity as that of the preferred catalysts shown in the other figures. In particular, the configuration of the binaphthyl ligand provides for one of the naphthyl groups to be above the plane of the catalyst and the other naphthyl group to be below the plane of the catalyst, thereby favoring approach to the oxygen atom from one side.

### The Third Group of Catalysts

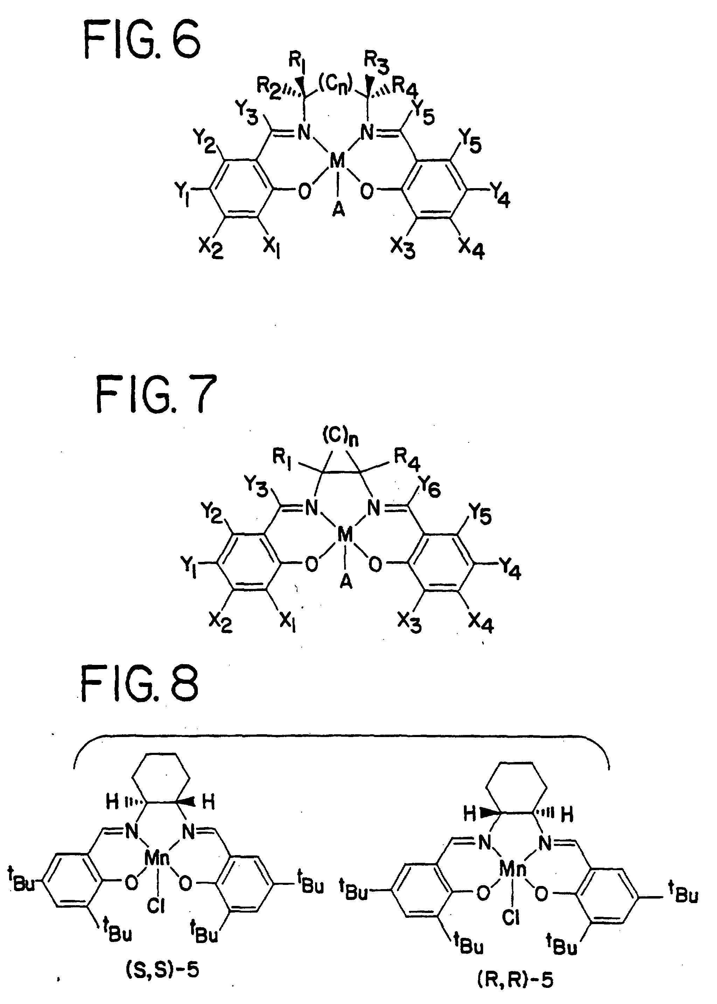

FIGURE 6 shows the structure of the third group. In accordance with the third group, the chiral catalyst has the following structure:

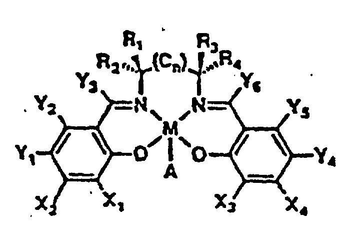

As with the first and second groups, M is a transition metal ion selected from the group mentioned above, with Mn being the most preferred.

Likewise, A is an anion selected from the group mentioned above, with Cl being most preferred.

Also, n can be 0, 1, or 2, but 0 is the most preferred.

As with the first and second groups, there is a blocking substituent on either X1 or X2 or on both. This blocking substituent is selected from the group consisting of aryls, primary alkyls, secondary alkyls, tertiary alkyls, and hetero atoms. There is also a blocking substituent selected from the same group on either X3 or X4 or on both. Preferably, the blocking substituents are at X1 and X3. More preferably they are the same group, and most preferably the blocking substituents are tert-butyl.

As a point of difference with the first group, the third group requires a blocking substituent located at the following positions: at least one of Y1 and Y2, and at least one of Y4 and Y5. These blocking substituents are selected from the group as those for X1-X4, namely the group consisting of aryls, primary alkyls, secondary alkyls, tertiary alkyls, and hetero atoms. The importance of these "side" blocking substituents will be discussed below.

In this third group, substituents Y3 and Y6 are independently selected from the group consisting of hydrogen and primary alkyl groups. Preferably, Y3 and Y6 are hydrogen.

Also in this third group, at least one of R1, R2, R3 and R4 is hydrogen. Where R1 is hydrogen, R3 is a primary alkyl. Where R2 is hydrogen, R4 is a primary alkyl. Where R3 is hydrogen, R1 is a primary alkyl. Finally, where R4 is hydrogen, R2 is a primary alkyl. Preferably, R1 and R4 are both hydrogen and R2 and R3 are primary alkyls. Most preferably, R2 and R3 are methyl groups.

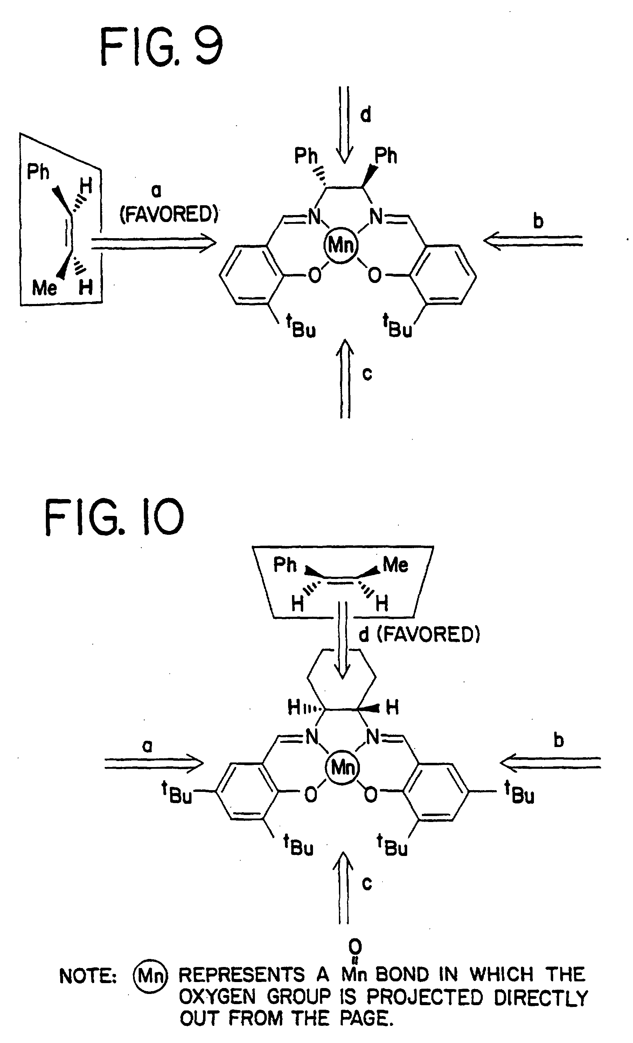

As can be seen, the catalyst of this third group is similar to the catalyst of the first group with the exception that the third group requires blocking substituents at the side positions of the catalyst, i.e. on the Y1 and/or Y2, and Y4 and/or Y5 sites. Also, either one or two of R1, R2, R3 and R4 is required to be an hydrogen, with the remaining substituents at the R sites required to be primary alkyls in the defined arrangement. The importance of this configuration and the proposed mechanism for the second catalyst are discussed below in connection with FIGURE 10.

### The Fourth Group of Catalysts

FIGURE 7 shows the structure of a catalyst of the fourth group. This catalyst has the following structure:

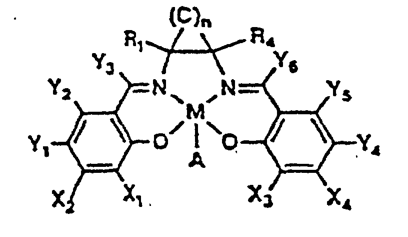

In this catalyst, the transition metal, M, and the anion, A, are selected from the same groups as above, with the same preferences.

Likewise, the substituents at X1, X2, X3, X4, Y1, Y2, Y4, and Y5 are selected from the same groups as in the second catalyst described above with the same preferences. In other words, this embodiment requires blocking substituents at the "bottom" and "sides" as does the second catalyst. Most preferably, X1, X3, Y1, and Y4 are all t-butyl.

The requirements and preferences for Y3 and Y6 are the same as with the third group. Preferably, Y3 and Y6 are hydrogen.

As can be seen, this catalyst of the fourth group includes a ring attached to the two nitrogen atoms, which ring is n+2 carbons long. In this catalyst, n can be 3, 4, 5 or 6. The carbons in the "Cₙ" portion can have substituents selected from hydrogen, alkyl, aryl, and hetero atoms. Preferably, the substituents on the carbons in the "Cₙ" portion are hydrogen.

In this fourth group, R1 and R4 are configured so as to be trans to each other. Also, R1 and R4 are selected from the group consisting of primary alkyls and hydrogen. Preferably, R1 and R4 are the same. Most preferably, both R1 and R4 are hydrogen.

Conceptually, the carbons in the ring which are adjacent the carbons which in turn are adjacent the nitrogen atoms are attached to what were shown as the R2 and R3 sites in the third group (FIGURE 6). Thus, this fourth group is, in some respects, a subset of the third group with the two ends of the n carbon chain (a primary alkyl) being attached to the R2 and R3 sites.

One distinction between the third and fourth groups is that the catalyst of the fourth group has R1 and R4 which can be either hydrogen or a primary alkyl.

FIGURE 8 shows a preferred catalyst of this fourth group used in the method of the invention. As can be seen in this catalyst, the ring is six-membered, that is, n=4. Also, R1 and R4, which are trans to each other, are hydrogen. X1, X3, Y1, and Y4 are all t-butyl. All other substituents are hydrogen.

FIGURES 9 and 10 illustrate the distinction between the mechanism proposed for the first and second groups and the proposed mechanism for the third and fourth groups.

FIGURE 9, representing the first and second groups shows the proposed favored approach of the prochiral olefin. Approach c is believed to be disfavored by the bulky t-butyl groups. Approach d is similarly unfavorable, due to the steric bulk of the phenyl groups on the catalyst. Approaches a and b are differentiated by the dissymmetry of the catalyst. As shown in the depicted embodiment, because the phenyl to the left is below the page and the phenyl to the right is above the page, it is predicted that approach b will be less favorable due to steric interactions between the olefin and the phenyl group. In the context of the more favored approach from the left (approach a), it is predicted that the more favorable approach of the olefin to the oxo group is such that the larger substituent on the olefin is oriented away from the t-butyl groups on the catalyst.

FIGURE 10, representing the third and fourth groups shows the proposed favored approach of the olefin when the catalyst has side blocking groups. It is believed that, because of the side t-butyl groups at Y1 and Y4, the approaches a from the left and b from the right are disfavored. likewise, because of the bottom blocking groups at X1 and X3, the approach c from the bottom is also disfavored. Thus, approach d from the top is favored. In addition, because of the chirality of the catalyst, the orientation of the prochiral olefin is also influenced. As shown in this depicted embodiment, because of greater steric hindrance on the right, the olefin is predicted to orient itself with the larger group on the left.

Because approach d is theorized to be the favored approach, the groups at R1 and R4 are limited to hydrogen and primary alkyls. In other words, it is believed that larger groups would block the approach d.

It should be noted that, although the above discussion is consistent with the observed results, the proposed mechanism for all four groups of catalysts is only theorized at this point. Consequently, the explanation is not to be viewed as limiting the scope of the invention as defined in the appended claims.

The preferred route to prepare the chiral catalysts used in the present invention is a condensation reaction with the substituted salicylaldehyde and the substituted diamine. In general, quantities of these compounds are reacted in a 2 to 1 molar ratio in absolute ethanol. The solutions are refluxed typically for I hour, and the salen ligand is either precipitated in analytically pure form by addition of water, or the metal complex is generated directly by addition of the metal as its acetate, halide, or triflate salt.

The following procedure is general for the preparation of:

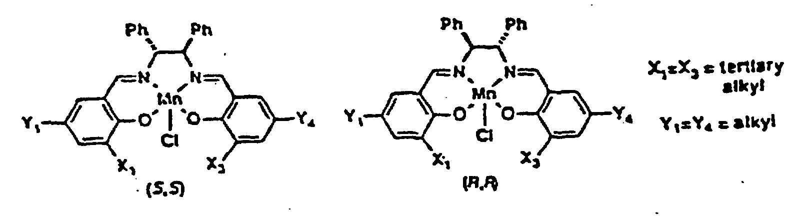

The salen ligand is redissolved in hot absolute ethanol to give a 0.1 M solution. Solid Mn(OAc)₂•4H₂O (2.0 equivalents) is added in one portion and the solution is refluxed for 1 h. Approximately 3 equivalents of solid LiCl are then added and the mixture is heated to reflux for an additional 0.5 h. Cooling the mixture to 0°C affords the Mn(III) complex 1 as dark brown crystals which are washed thoroughly with H₂O and isolated by filtration in ≈75% yield. An additional crop of material can be obtained by dropwise addition of H₂O to the mother liquor. Combined yields of catalyst are 89-96% for this step, and 81-93 % overall from the optically pure 1,2-diphenylethylene diamine. Acceptable C, H, N, Cl, and Mn analyses of each of the catalysts have been obtained (±0.4%), although these vary according to the extent of water and ethanol incorporation in the powdery product. Enantioselectivities in the epoxidation reactions did not vary among different batches of a given catalyst, indicating that the solvent content of the catalysts does not influence its effectiveness.

Another example of the method of preparing the catalyst is described as follows: Most preferably, the starting diamine is R,R- or S,S-1,2-diamino-1,2-diphenylethane and the starting salicylaldehyde is 3-tert-butyl-salicylaldehyde.

A solution of 2.0 mmol of 3-tert-butylsalicylaldehyde in 3 ml of absolute ethanol is added dropwise to a solution of 1.0 mmol of (R,R)-1,2-diamino-1,2-diphenylethane in 5 ml of ethanol. The reaction mixture is heated to reflux for 1 h and then 1.0 mmol of Mn(OAc)₂•4H₂O is added in one portion to the hot (60°C) solution. The color of the solution immediately turns from yellow to brown upon addition. It is refluxed for an additional 30 min and then cooled to room temperature. A solution of 10% NaCl (5ml) is then added dropwise and the mixture stirred for 0.5h. The solvents are then removed in vacuo and the residue is triturated with 50 ml of CH₂Cl₂ and 50 ml of H₂O. The organic layer is separated and the brown solution was washed with saturated NaCl. Separation of the organic phase and removal of solvent resulted in a crude material which was recrystallized from C₆H₆/C₆H₁₄ to give 0.938 mmol of the (R,R)-catalyst shown above (93.8%).

In accordance with the epoxidation method aspect of the invention, the chromene derivative, an oxygen atom source, and the chiral catalyst described in the method according to claim 1 are reacted under such conditions and for such time as is needed to epoxidize said chromene derivative.

Preferably, the chromene derivative to be epoxidized is 2,2-dimethylchromene.

The oxygen atom source used in the epoxidation reaction should be an oxidant which is relatively unreactive toward olefins under mild conditions. Preferably, the oxygen atom source is selected from the group consisting of NaOCl, iodosylmesitylene, NaIO₄, NBu₄IO₄, potassium peroxymonosulfate, magnesium monoperoxyphthalate, and hexacyanoferrate ion. More preferably, the oxygen atom source is selected from the group consisting of NaOCI and iodosylmesitylene. For economic reasons, the most preferred oxygen atom source is NaOCl.

A preferred method uses NaOCl as the oxygen atom source. For convenience this method will be designated METHOD A. The details of METHOD A in which only for reasons of illustration cis-β-methylstyrene is used as an olefin are as follows:

A solution of 0.05 M Na₂B₄O₇ 10H₂O (1.0ml) is added to a 2.5 ml solution of undiluted commercial household bleach (Chlorox). The pH of the resulting buffered solution is approximately 9.5, and it is adjusted to a pH of about 10.5 by addition of a few drops of 1 M NaOH solution. To this solution is added a solution of 0.02 mmol of the preferred catalyst and 1.0 mmol of cis B methylstyrene in 2.0 ml of CH₂Cl₂. The two-phase mixture is stirred at room temperature and the reaction progress is monitored by capillary gas chromatography. After approximately 3 hours, 10 ml of CH₂Cl₂ is added to the mixture and the brown organic phase is separated, washed twice with 10 ml H₂O and once with 10 ml saturated NaCl solution, and then dried for 15 minutes over anhydrous Na₂SO₄. The solution is filtered and solvent is removed under vacuum. The residue is purified by flash chromatography on silica gel using a 20:80 mixture of CH₂Cl₂:hexane as the eluting solvent. Pure epoxide is isolated as a colorless liquid in 70% yield (0.70 mmol) by combination of the product-containing fractions and removal of solvent under vacuum. The optical purity of this material is determined to be 85% ee by the method described below.

In a slightly less preferred embodiment, iodosylmesitylene is used as the oxygen atom source. For convenience, this method is designated as METHOD B and has the following preferred details: A solution of 1.0 mmol of olefin, 8 ml CH₂Cl₂ and 0.04 mmol of the catalyst are stirred at room temperature as solid iodosomesitylene is added in 0.3 mmol portions at 15-30 minute intervals. Disappearance of starting olefin is complete after addition of 6 portions (1.8 mmol) of total iodosylmesitylene. Solvent is removed in vacuo, the residue is extracted with hexane, and the mixture was filtered through Celite to remove catalyst and other solids. Pure epoxide was obtained by flash chromatography (10g SiO₂, CH₂Cl₂/hexane 20:80 eluent). Enantiomeric excesses are determined by 1H NMR using Eu(hfc)₃ as a chiral shift reagent, or in the case of stilbene oxide by direct separation by HPLC on a commercial (Regis) covalently-bound leucine Pirkle column. Absolute configurations were assigned by comparison of αD with accepted literature values.

An alternative method also uses a pyridine-N-oxide derivative as a coordinating ligand, in addition to NaOCl as the oxygen source. More preferably, 4-phenylpyridine-N-oxide or 4-t-butylpyridine-N-oxide is used. Even more preferably, 4-phenylpyridine-N-oxide is used.

The trans-epoxide is a significant (about 25%) by-product of the epoxidation reaction. Preferably, the mixture of diastereomeric products is enriched in the desired cis- form by flash chromatography. Even more preferably, for large scale batches, chromatography is not performed.

The next steps are shown in Scheme 2. 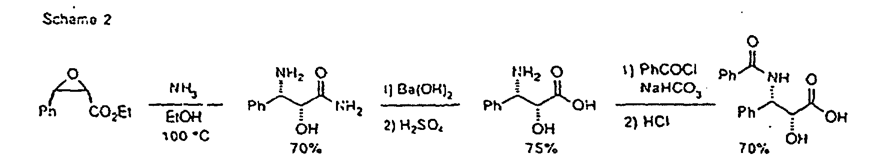 The epoxide mixture is reacted with ammonia in ethanol, which results in regioselective ring-opening to the desired 3-phenyl-isoserine amide derivative. Preferably, very little regioisomer is detected in the crude amide mixture by ¹H NMR. Next, diastereomerically pure 3-phenyl-isoserinamide is isolated by recrystallization of the crude product mixture. For convenience this method will be designated METHOD C. The details of METHOD C are as follows:

A solution of 0.05 M Na₂B₄O₇ • 10H₂O (1.0ml) or other suitable buffer such as phosphate is added to a 2.5 ml solution of undiluted commercial household bleach (Chlorox® ). The pH of the resulting buffered solution is approximately 9.5, and it is adjusted to a pH of about 10.5-11.5 by addition of a few drops of 1 M NaOH solution and cooled in an ice bath to about 0-4° C. A separate solution of 10 mmol of alkene and 2.0 mmol (or 20 mol%) of a pyridine-N-oxide derivative are dissolved in 10 ml of CH₂Cl₂. Next, 0.05-0.6 mmol (0.5-6 mol%) of catalyst 1 were added to the alkene solution and 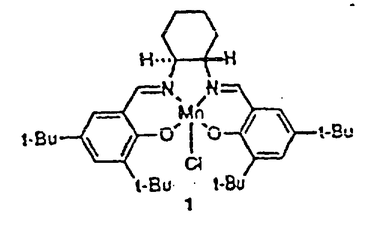 cooled separately in an ice bath. When the two solutions were at 0-4° C, they were combined, and the two-phase mixture was stirred. The reaction progress was monitored by capillary gas chromatography. After about one to five hours, 200 ml of hexane was added to the mixture and the organic phase was separated, washed twice with 100 ml H₂O and once with 100 ml saturated NaCI solution, and then dried for 15 minutes over anhydrous Na₂SO₄. The solution is filtered and solvent is removed under vacuum. The residue is purified by chromatography, distillation or crystallization. Pure epoxides were isolated, and the optical purity of the materials were determined as described in more detail below.

### Method of Chromene Epoxidation

As noted above, the present invention is a method of using a chiral catalyst to epoxidize a chromene derivative, thus producing an epoxychroman. The structure of the chromene derivative is as follows: 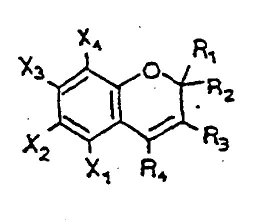 wherein R1, R2, R3, R4, X1, X2, X3, and X4 are each selected from the group consisting of hydrogen, aryls, primary alkyls, secondary alkyls, tertiary alkyls, and hetero atoms, and wherein no more than one of R1 and R2 are hydrogen.

It has been found that when both R1 and R2 are hydrogen, i.e. when the chromene is not substituted at the R1 and R2 locations, the epoxide is not formed (see Example 9 below).

Preferably, R1 and R2 are the same group. In this situation, the chromene derivative is prochiral.

Also, the chromene derivative preferably includes an alkyl group at both R1 and R2. More preferably, the chromene derivative includes a methyl group at R1 and R2. Most preferably, the chromene derivative is 6-cyano-2,2-dimethylchromene, namely the precursor for making cromakalim. As mentioned above, this most preferred chromene derivative can be epoxidized with a remarkably high degree of enantioselectivity to the epoxychroman useful in producing enantiomerically pure cromakalim (see Example 2 below).

As noted above, this embodiment of the present invention has been found to produce remarkable enantioselectivity in the epoxidation of chromene derivatives. In addition, the catalysts used in the present invention have been found to provide remarkably high yields (See Examples 2-8 and 10 below). The catalyst of the fourth group (FIG. 8) above is the most preferred catalyst used in the present method. Experiments have shown that when a racemic mixture of the chiral catalysts are used in the reaction that relatively high yields of a racemic mixture of the epoxychromans are achieved. Consequently, in accordance with a less preferred embodiment of the invention, when a racemic mixture of the epoxychromans is desirable or acceptable, a racemic mixture of the chiral catalyst is used with the chromene derivatives. Nevertheless, because enantiomerically pure epoxychromans are typically highly desirable, particularly as synthetic precursors, enantiomerically pure chiral catalysts are clearly preferred.

In accordance with the epoxychroman synthetic method of the present invention, the chromene derivative, an oxygen atom source, and the chiral catalyst are reacted under such conditions and for such time as is needed to epoxidize said chromene derivative. Alternatively, a pyridine-N-oxide derivative is added to the reaction mixture.

The oxygen atom source used in the epoxidation reaction should be an oxidant which is relatively unreactive toward olefins under mild conditions. Preferably, the oxygen atom source is selected from the group consisting of NaOCI, iodosylmesitylene, NaIO₄, NBu₄IO₄, potassium peroxymonosulfate, magnesium monoperoxyphthalate, H₂O₂, peroxybenzoic acid derivatives, and hexacyanoferrate ion. More preferably, the oxygen atom source is selected from the group consisting of NaOCl and iodosylmesitylene. For economic reasons, the most preferred oxygen atom source is NaOCl.

In the most preferred method for chromene epoxidation, NaOCI is the oxygen atom source, as described above for METHOD A. In a slightly less preferred embodiment, iodosylmesitylene is used as the oxygen atom source, as described above for METHOD B. Alternatively, a pyridine-N-oxide derivative and NaOCl arc used, as described in METHOD C.

### EXAMPLES

The following examples are provided by way of expianation and illustration. As such, these examples are not to be viewed as limiting the scope of the invention as defined by the appended claims.

### Preparation of the Catalysts

### Procedure for the preparation of the most preferred catalyst of the fourth group of catalysts used in the method of the present invention

### (R,R)- and (S,S)-1,2,-bis(3,5-di-tert-butylsalicylide-amino)cyclohexane

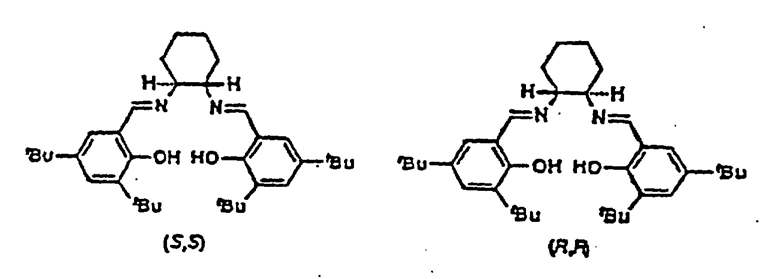

3,5-Di-t-butylsalicylaldehyde (2.0 equivalents) was added as a solid to a 0.2 M solution of (R,R) or (S,S) 1,2- diaminocyclohexane (1.0 equivalent) in absolute ethanol. The mixture was heated to reflux for 1 hr. and then H₂O was added dropwise to the cooled bright yellow solution. The resulting yellow crystalline solid was collected by filtration and washed with a small portion of 95% ethanol. The yield of analytically pure salen ligand obtained in this manner was 90-97%.

Spectroscopic and analytical data for the salen ligand: ¹H NMR (CDCl₃) δ 13.72 (s, 1H), 8.30 (S, 1H), 7.30 (d, J = 2.3 Hz, 1H), 6.98 (d, J = 2.3 Hz, 1H), 3.32 (m, 1H), 2.0-1.8 (m, 2H), 1.8-1.65 (m, 1H), 1.45 (m, 1H), 1.41 (s, 9H), 1.24 (s, 9H). ¹³C NMR (CDCl₃): δ 165.8, 158.0, 139.8, 136.3, 126.0, 117.8, 72.4, 34.9, 33.0, 31.4, 29.4, 24.3. Anal. Calcd for C₃₆H₅₄N₂O₂: C, 79.07; H, 9.95; N, 5.12. Found: C, 79.12; H, 9.97; N, 5.12.

### (R,R)- and (S,S)-[1,2-bis(3,5-di-tert-butylsalicylide-amino)cyclohexane]-manganese(III) chloride.

The salen ligand immediately above is redissolved in hot absolute ethanol to give a 0.1 M solution. Solid Mn(OAc)₂•4H₂O(2.5 equivalents) is added in one portion and the solution is refluxed for 1 hr. Approximately 5 equivalents of solid LiCl are then added and the mixture is heated to reflux for an additional 0.5 hr. Cooling the mixture to 0°C and addition of a volume of water equal to the volume of the brown ethanolic solution to afford the Mn(III) complex as a dark brown powder which are washed thoroughly with H₂O, and isolated by filtration in 81-93% yield. Acceptable C, H, N, Cl, and Mn analyses of the catalyst have been obtained (±0.4%), but these vary according to the extent of water and ethanol incorporation in the powdery product. Enantioselectivities in the epoxidation reactions are invariant with different batches of a given catalyst, indicating that the solvent content of the catalyst does not influence its effectiveness.

Analytical data for this catalyst: Anal. Calcd for C₃₆H₅₂ClMnN₂O₂•C₂H₅OH: C, 67.19; H, 8.31; Cl, 5.22; Mn, 8.09; N, 4.12; Observed: C, 67.05; H, 8.34; Cl, 5.48; Mn, 8.31; N, 4.28.

### Procedures for the Asymmetric Epoxidation of Chromene Derivatives

### Method A (NaOCl as oxygen atom source):

A solution of 0.05 M Na₂B₄O₇•10H₂O (1.0 ml) was added to a 2.5 ml solution of undiluted commercial household bleach (Clorox® ). The pH of the resulting buffered solution was approximately 9.5, and it was adjusted to a pH of 10.5 by addition of a few drops of 1 M NaOH solution. To this solution. was added a solution of about 0.005 to 0.02 mmol of the catalyst and about 1.0 mmol of olefin in 2.0 ml of CH₂Cl₂. The two-phase mixture was stirred at room temperature and the reaction progress was monitored by capillary gas chromatography. Reactions were complete within approximately 1-5 hours. After the reaction was complete, 10 ml of CH₂Cl₂ was added to the mixture and the brown organic phase was separated, washed twice with 10 ml H₂O and once with 10 ml saturated NaCl solution, and then dried for 15 min over anhydrous Na₂SO₄. The solution was filtered and solvent was removed under vacuum. The residue was purified by standard procedures using flash chromatography on 10g of silica gel using a mixture of CH₂Cl₂/hexane as the eluting solvent. Pure epoxide was isolated by combination of the product-containing fractions and removal of solvent under vacuum. Enantiomeric excesses were determined by ¹H NMR using Eu(hfc)₃ as a chiral shift reagent, or in the case of stilbene oxide by direct separation by HPLC on a commercial (Regis) covalently-bound leucine Pirkle column. Absolute configurations were assigned by comparison of [α]^{D} with accepted literature values.

### Method B (iodosylmesitylene as oxygen atom source)

A solution of 1.0 mmol of olefin, 8 ml CH₂Cl₂ and 0.04-0.08 mmol of the catalyst was stirred at room temperature as solid iodosomesitylene was added in 0.3 mmol portions at 15-30 minute intervals. Disappearance of starting olefin was complete after addition of 4-10 portions (1.2 to 3 equivalents) of total iodosylmesitylene. Solvent was removed in vacuo, the residue was extracted with hexane, and the mixture was filtered through Celite diatomaceous earth to remove catalyst and other solids. Pure epoxide was obtained by flash chromatography (10g SiO₂, CH₂Cl₂/hexane eluent). The optical purity of this material was determined by the method described above.

### Asymmetric Epoxidation of a Representative Chromene Derivative with a Catalyst from the fourth group

### Example 1

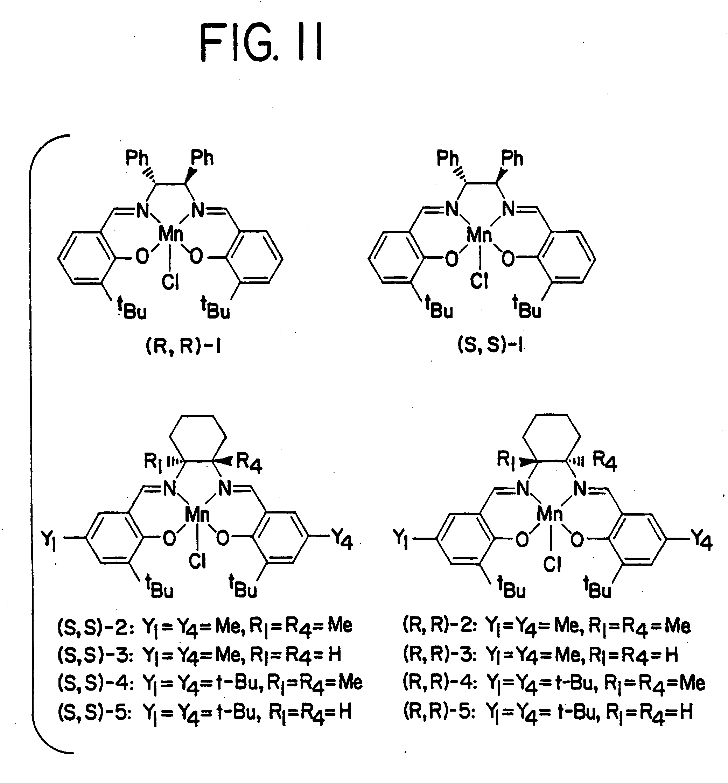

The key to the catalyst numbering system is found in FIGURE 11. The catalyst used in Example 1 being the most preferred catalyst of the fourth group. It is also noted that Example 1 was run with method B described above.

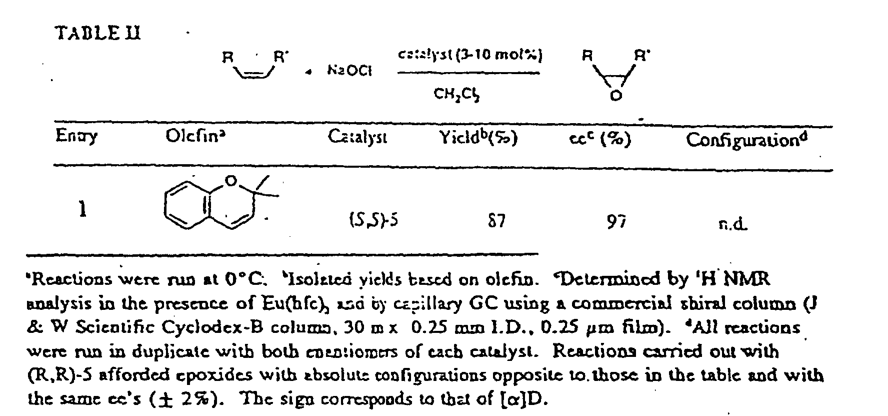

As shown in Example 1, the most preferred catalyst of the fourth group catalyzes the epoxidation of chromene derivatives with excellent enantioselectivity.

### Examples 2-9 Epoxidation of Chromene Derivatives

The following Examples 2-9 were carried out to show the effectiveness of the present method to enantioselectively epoxidize various chromene derivatives. The catalyst used in these examples is the R,R enantiomer shown in Figure 8. The method described above as Method A was used for these examples:

The results are shown in Table III. It is noted that the unsubstituted chromene in Example 9 did not produce an epoxychroman.

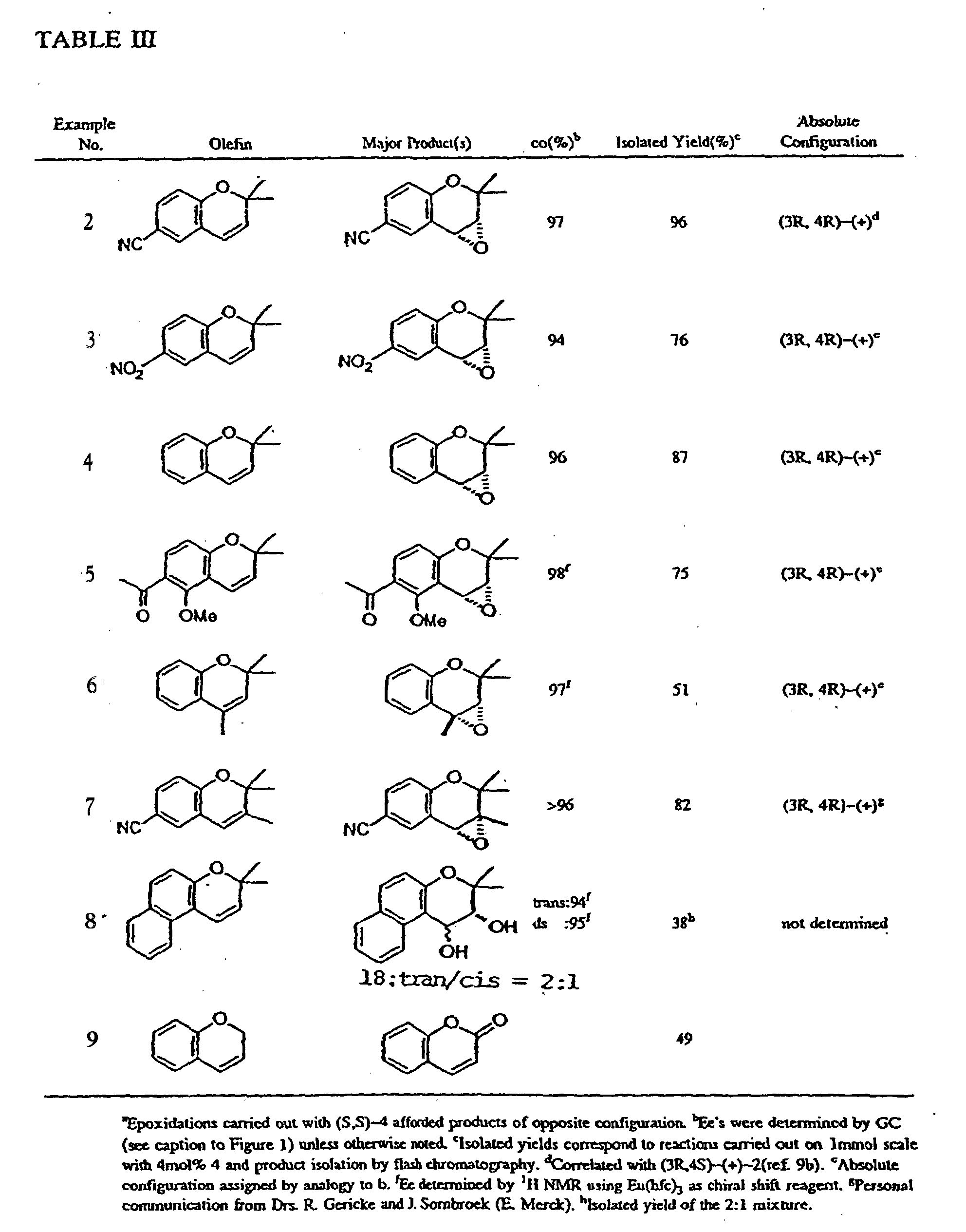

### Example 10

Example 10 was carried out as a larger scale production of the epoxychroman produced in Example 2 above, namely 6-cyano-2,2-dimethyl-3,4-epoxychroman.

The pH of a solution of commercial household bleach (Clorox® ) was buffered to pH=11.3 with 0.05 M Na₂HPO₄ and 1 N NaOH and then cooled to O °C. To 500 ml of this solution (approximately 0.55 M in NaOCl) was added a 0 °C solution of 6-cyano-2,2-dimethylchromene and the catalyst (3.1 g, 5.0 mmol, 3.7 mol%) in 135 ml of CH₂Cl₂. The two-phase system was mechanically stirred at 0 °C and the reaction progress was monitored by HPLC. After 9 hours, the heterogeneous brown mixture was filtered through a pad of Celite diatomaceous earth and the organic phase was separated, washed once with 500 ml saturated NaCl solution, and then dried (Na₂SO₄). The ee of the crude product obtained after solvent removal was determined for each example by GC analysis. The brown oily residue was then dissolved in 200 ml of boiling absolute ethanol and then water (200 ml) was added slowly to the hot solution. A hot gravity filtration afforded a pale yellow solution from which the crystallized epoxychroman was isolated. This isolated yield was 81% and the ee was measured at 99% by GC analysis.

### Example 11

Example 11 was carried out to produce the cromakalin compound shown in Figure 13. The epoxychroman produced in Example 10, namely 6-cyano-2,2-dimethy-3,4-epoxychroman, (1g, 4.97 mmol), 3-hydroxy-1-methyl-1,6-dihydropyridazin-6-one (0.652 g, 5.17 mmol) and pyridine (0.491 g, 6.21 mmol) were refluxed together in ethanol (10 ml) for 8 hours. The homogenous yellow mixture was then concentrated under vacuum and the residue was isolated by flash chromatography on 70 g of silica and ethyl acetate as eluent. The isolated yield was 1.38 g (85%).

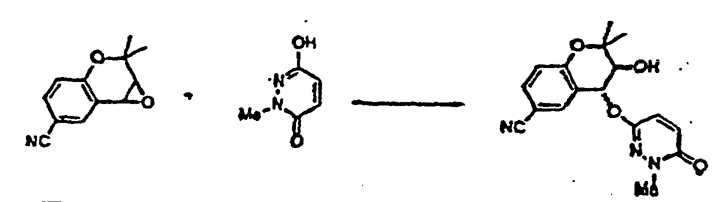

### Example 12

Example 12 was carried out to produce the cromakalin shown in Figure 14. Sodium hydride (0.199 g of a 60% suspension in mineral oil, 4.97 mmol) was suspended in DMSO (1.5 ml) and 2-pyrrolidinone (0.423 g, 4.97 mmol) was added to the stirred mixture at room temperature under a dry nitrogen atmosphere. The epoxychroman from Example 10 (1g, 4.97 mmol) was then added as a solid to the grey foamy mass. The mixture was stirred at room temperature for 10 hours. The orange-red mixture was then treated with 10 ml of water and the resulting thick yellow precipitated was extracted 5 times with 10 ml of ethyl acetate. Removal of solvent and chromatography on silica (100g, ethyl acetate eluent) afforded pure product which was recrystallized from ethyl acetate. This isolated yield was 0.808 g (56%).

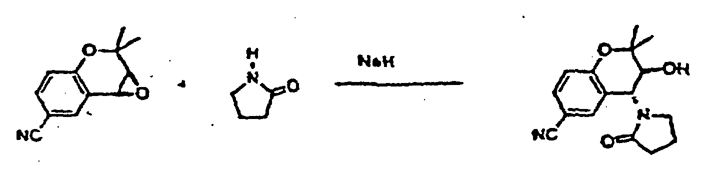

### Examples 13 and 14

### Effect Of Pyridine-N-Oxide Derivative On Epoxidation

The two alkenes shown in Table IV below were epoxidized with the presence of a pyridine-N-oxide derivative in the following manner.

A solution of 10 mmol of an alkene and 2.0 mmol (20 mol %) of a pyridine-N-oxide derivative were dissolved in 10 ml of CH₂Cl₂. Either 4-phenylpyridine-N-oxide (A in the table) or 4-t-butylpyridine-N-oxide (B in the table) was used.

Then, 0.08-1.0 mmol (0.8-10 mol%) of Catalyst 1 (see below) were added to the alkene solution. The table shows the amounts of catalyst used in each example. This solution and buffered bleach solution (pH=11.25) were cooled separately in an ice bath and then combined at 0-4° C. This two-phase mixture was stirred for one to five hours. Then, 200 ml of hexane was added to the solution, and the organic phase was separated and washed once with 100 ml water and once with 100 ml brine. The organic phase was then dried over Na₂SO₄. The solvent was removed under vacuum. The residue was subjected to purification distillation but could also be purified by chromatography or crystallization. The enantiomeric compositions of the epoxide were established by GC on a chiral capillary column and by ¹H NMR with a chiral shift reagent (Eu(hfc)₃).

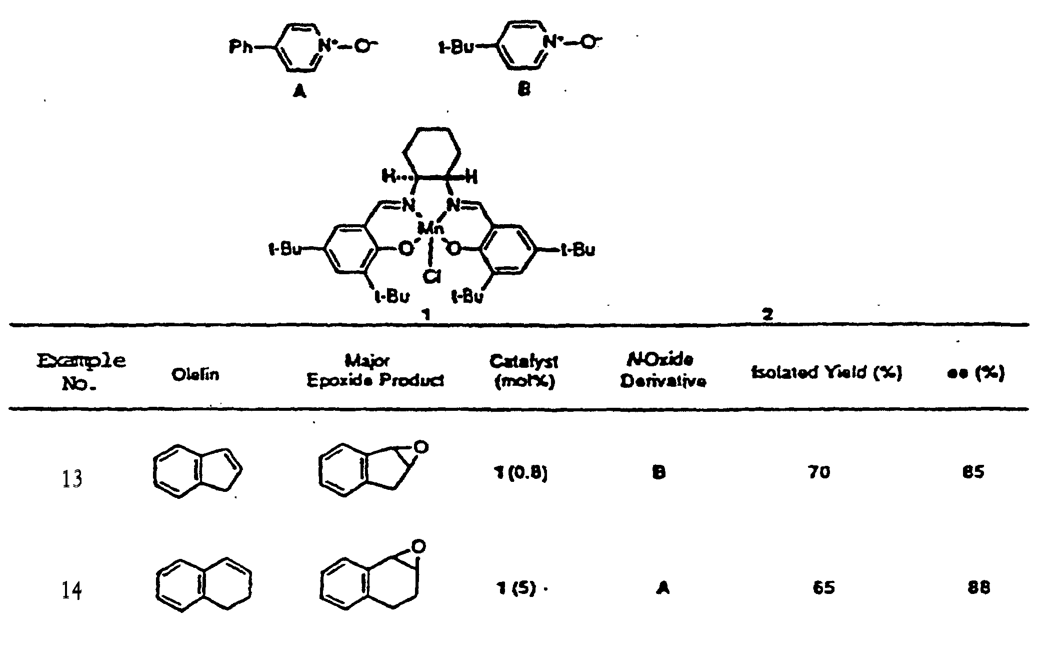

## Claims (Claims for the following Contracting State(s): AT, BE, CH, DE, DK, ES, FR, GB, GR, IT, LU, NL, SE)

1. A method of enantioselectively epoxidizing a chromene derivative to an epoxychroman with a chiral catalyst comprising the steps of:
oxidizing a chromene derivative having the general formula: 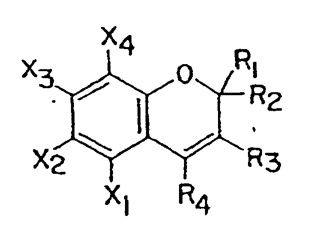
wherein R1, R2, R3, R4, X1, X2, X3 and X4 are each selected from the group consisting of hydrogen, aryls, primary alkyls, secondary alkyls, tertiary alkyls, and hetero atoms, and wherein no more than one of R1 and R2 are hydrogen;
with an oxygen atom source; in the presence of a chiral catalyst having the general formula: 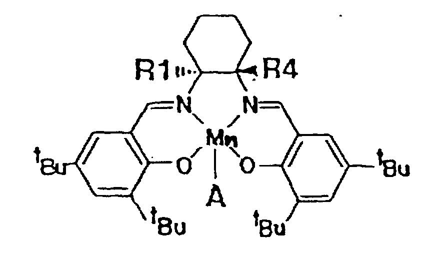
where A is an anion and
where R1 and R4 are trans to each other and at least one of R1 and R4 is selected from the group consisting of primary alkyls and hydrogen,
with the proviso that the chromene derivative is not 2,2-dimethylchromene when the anion A of the catalyst is Cl and R1 = R4 = H or either R1 or R4 or both are primary alkyls.

2. The method of claim 1, wherein the opposite enantiomer of the chiral catalyst is included to thereby produce a racemic mixture of the epoxychroman.

3. The method of claims 1 or 2, further comprising the step of adding a pyridine-N-oxide to the reaction mixture.

4. The method according to any of claims I to 3, wherein the chromene derivative is 6-cyano-2,2-dimethylchromene.

5. The method according to any of claims 1 to 4, wherein the anion A is selected from the group consisting of PF₆, (aryl)₄, BF₄, B(aryl)₄, halide, acetate, triflate and tosylate.

## Claims (Claims for the following Contracting State(s): IE, MC)

1. A method of enantioselectively epoxidizing a chromene derivative to an epoxychroman with a chiral catalyst comprising the steps of:
oxidizing a chromene derivative having the general formula: 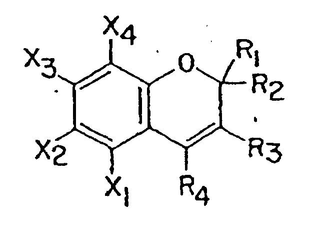
wherein R1, R2, R3, R4, X1, X2, X3 and X4 are each selected from the group consisting of hydrogen, aryls, primary alkyls, secondary alkyls, tertiary alkyls, and hetero atoms, and wherein no more than one of R1 and R2 are hydrogen;
with an oxygen atom source; in the presence of a chiral catalyst having the general formula: 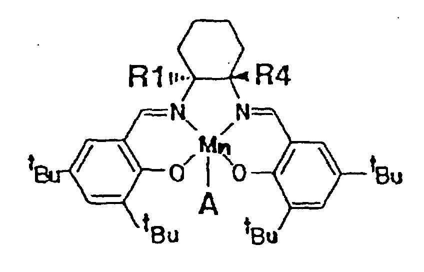
where A is an anion and
where R1 and R4 are trans to each other and at least one of R1 and R4 is selected from the group consisting of primary alkyls and hydrogen.

2. The method of claim 1, wherein the opposite enantiomer of the chiral catalyst is included to thereby produce a racemic mixture of the epoxychroman.

3. The method of claims 1 or 2, further comprising the step of adding a pyridine-N-oxide to the reaction mixture.

4. The method according to any of claims I to 3, wherein the chromene derivative is 6-cyano-2,2-dimethylchromene.

5. The method according to any of claims 1 to 4, wherein the anion A is selected from the group consisting of PF₆, (aryl)₄, BF₄, B(aryl)₄, halide, acetate, triflate and tosylate.

## Patentansprüche (Patentansprüche für folgende(n) Vertragsstaat(en): AT, BE, CH, DE, DK, ES, FR, GB, GR, IT, LU, NL, SE)

1. Ein Verfahren zur enantioselektiven Epoxidierung eines Chromenderivats zu einem Epoxychroman mit einem chiralen Katalysator, umfassend die Schritte:
Oxidieren eines Chromenderivats der allgemeinen Formel 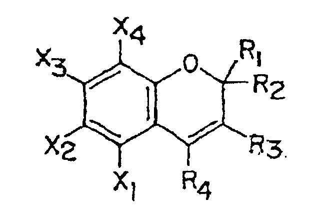
worin R₁, R₂, R₃, R₄, X₁, X₂, X₃ und X₄ jeweils aus der Gruppe bestehend aus einem Wasserstoffatom, Aryl-, primären Alkyl-, sekundären Alkyl-, tertiären Alkylgruppen und Heteroatomen ausgewählt sind, wobei nicht mehr als einer der Reste R₁ und R₂ ein Wasserstoffatom ist;
mit einer Sauerstoffatom-Quelle in Gegenwart eines chiralen Katalysators der allgemeinen Formel 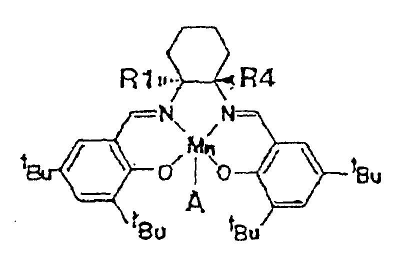
worin A ein Anion und
worin R1 und R4 trans zueinander angeordnet sind und mindestens einer der Reste R1 und R4 aus der Gruppe bestehend aus primären Alkylgruppen und Wasserstoffatomen ausgewählt ist, mit der Maßgabe, dass das Chromenderivat nicht 2,2-Dimethylchromen ist, wenn das Anion A des Katalysators Cl und R1 = R4 = H sind oder entweder R1 oder R4 oder beide primäre Alkylgruppen sind.

2. Verfahren nach Anspruch 1, bei dem das andere Enantiomer des chiralen Katalysators einbezogen wird, um ein racemisches Gemisch des Epoxychromans zu erzeugen.

3. Verfahren nach Anspruch 1 oder 2, das ferner den Schritt des Zugebens eines Pyridin-N-oxids zu dem Reaktionsgemisch umfasst.

4. Verfahren nach einem der Ansprüche 1 bis 3, bei dem das Chromenderivat 6-Cyano-2,2-dimethylchromen ist.

5. Verfahren nach einem der Ansprüche 1 bis 4, bei dem das Anion A aus der Gruppe bestehend aus PF₆, (Aryl)₄, BF₄, B(aryl)₄, Halogenid, Acetat, Triflat und Tosylat ausgewählt ist.

## Patentansprüche (Patentansprüche für folgende(n) Vertragsstaat(en): IE, MC)

1. Ein Verfahren zur enantioselektiven Epoxidierung eines Chromenderivats zu einem Epoxychroman mit einem chiralen Katalysator, umfassend die Schritte:
Oxidieren eines Chromenderivats der allgemeinen Formel 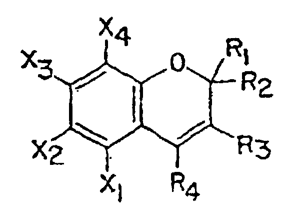
worin R₁, R₂, R₃, R₄, X₁, X₂, X₃ und X₄ jeweils aus der Gruppe bestehend aus einem Wasserstoffatom, Aryl-, primären Alkyl-, sekundären Alkyl-, tertiären Alkylgruppen und Heteroatomen ausgewählt sind, wobei nicht mehr als einer der Reste R₁ und R₂ ein Wasserstoffatom ist;
mit einer Sauerstoffatom-Quelle in Gegenwart eines chiralen Katalysators der allgemeinen Formel 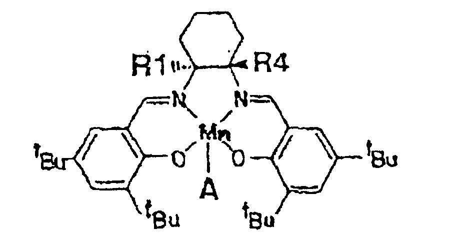
worin A ein Anion und
worin R1 und R4 trans zueinander angeordnet sind und mindestens einer der Reste R1 und R4 aus der Gruppe bestehend aus primären Alkylgruppen und Wasserstoffatomen ausgewählt ist.

2. Verfahren nach Anspruch 1, bei dem das andere Enantiomer des chiralen Katalysators einbezogen wird, um ein racemisches Gemisch des Epoxychromans zu erzeugen.

3. Verfahren nach Anspruch 1 oder 2, das ferner den Schritt des Zugebens eines Pyridin-N-oxids zu dem Reaktionsgemisch umfasst.

4. Verfahren nach einem der Ansprüche 1 bis 3, bei dem das Chromenderivat 6-Cyano-2,2-dimethylchromen ist.

5. Verfahren nach einem der Ansprüche 1 bis 4, bei dem das Anion A aus der Gruppe bestehend aus PF₆, (Aryl)₄, BF₄, B(aryl)₄, Halogenid, Acetat, Triflat und Tosylat ausgewählt ist.

## Revendications (Revendications pour l'(les) Etat(s) contractant(s) suivant(s): AT, BE, CH, DE, DK, ES, FR, GB, GR, IT, LU, NL, SE)

1. Procédé d'époxydation énantiosélective d'un dérivé de chromène en un époxychromanne avec un catalyseur chiral, comprenant les étapes de:
oxydation d'un dérivé de chromène ayant la formule générale: 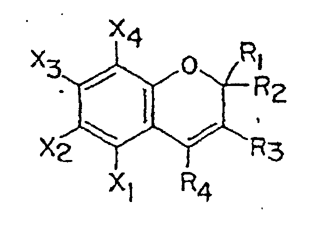
où R₁, R₂, R₃, R₄, X₁, X₂, X₃ et X₄ sont chacun choisis dans le groupe consistant en hydrogène, aryles, alkyles primaires, alkyles secondaires, alkyles tertiaires, et hétéroatomes, et où pas plus de l'un des R₁ et R₂ sont l'hydrogène;
avec une source d'atomes d'oxygène, en présence d'un catalyseur chiral ayant la formule générale: 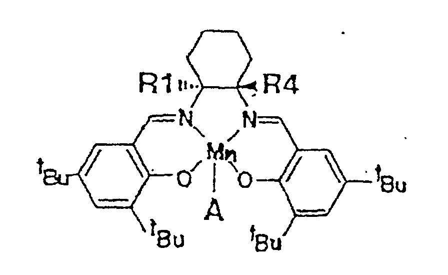
où A est un anion et
où R₁ et R₄ sont en trans l'un de l'autre et au moins l'un des R₁ et R₄ est choisi dans le groupe consistant en alkyles primaires et hydrogène,
avec comme condition que le dérivé de chromène n'est pas le 2,2-diméthyl-chromène quand l'anion A du catalyseur est Cl et R₁ = R₄ = H ou soit R₁, soit R₄, soit les deux sont des alkyles primaires.

2. Procédé selon la revendication 1, dans lequel l'énantiomère opposé du catalyseur chiral est indus pour produire ainsi un mélange racémique de l'époxychromanne.

3. Procédé selon les revendications 1 ou 2, comprenant en outre l'étape d'addition d'un N-oxyde de pyridine au mélange réactionnel.

4. Procédé selon l'une quelconque des revendications 1 à 3, dans lequel le dérivé de chromène est le 6-cyano-2,2-diméthylchromène.

5. Procédé selon l'une quelconque des revendications 1 à 4, dans lequel l'anion A est choisi dans le groupe consistant en PF₆, (aryle)₄, BF₄, B(aryle)₄, halogénure, acétate, triflate et tosylate.

## Revendications (Revendications pour l'(les) Etat(s) contractant(s) suivant(s): IE, MC)

1. Procédé d'époxydation énantiosélective d'un dérivé de chromène en un époxychromanne avec un catalyseur chiral, comprenant les étapes de:
oxydation d'un dérivé de chromène ayant la formule générale: 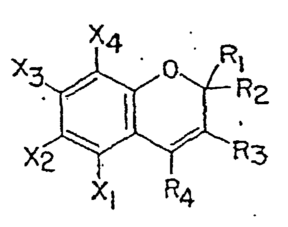
où R₁, R₂, R₃, R₄, X₁, X₂, X₃ et X₄ sont chacun choisis dans le groupe consistant en hydrogène, aryles, alkyles primaires, alkyles secondaires, alkyles tertiaires, et hétéroatomes, et où pas plus de l'un des R₁ et R₂ sont l'hydrogène;
avec une source d'atomes d'oxygène, en présence d'un catalyseur chiral ayant la formule générale: 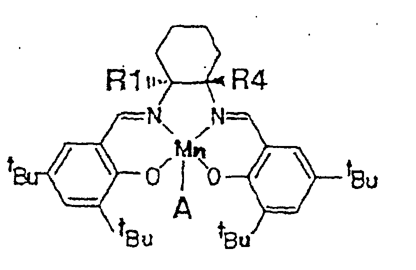
où A est un anion et
où R₁ et R₄ sont en trans l'un de l'autre et au moins l'un des R₁ et R₄ est choisi dans le groupe consistant en alkyles primaires et hydrogène.

2. Procédé selon la revendication 1, dans lequel l'énantiomère opposé du catalyseur chiral est inclus pour produire ainsi un mélange racémique de l'époxychromanne.

3. Procédé selon les revendications 1 ou 2, comprenant en outre l'étape d'addition d'un N-oxyde de pyridine au mélange réactionnel.

4. Procédé selon l'une quelconque des revendications 1 à 3, dans lequel le dérivé de chromène est le 6-cyano-2,2-diméthylchromène.

5. Procédé selon l'une quelconque des revendications 1 à 4, dans lequel l'anion A est choisi dans le groupe consistant en PF₆, (aryle)₄, BF₄, B(aryle)₄, halogénure, acétate, triflate et tosylate.
